(19) Europäisches Patentamt
European Patent Office
Office européen des brevets

(11) Veröffentlichungsnummer: **0 025 079**
**A1**

(12) # EUROPÄISCHE PATENTANMELDUNG

(21) Anmeldenummer: 80102316.9

(22) Anmeldetag: 28.11.78

(51) Int. Cl.³: C 07 C 59/68
C 07 C 69/712, C 07 C 79/35
A 01 N 31/16

(30) Priorität: 28.11.77 LU 78591
15.09.78 CH 9667/78

(43) Veröffentlichungstag der Anmeldung:
18.03.81 Patentblatt 81/11

(84) Benannte Vertragsstaaten:
BE CH DE FR GB LU NL SE

(60) Publication number of the earlier application in accordance with Art. 76 EPC: 0 002 246

(71) Anmelder: F. HOFFMANN-LA ROCHE & CO.
Aktiengesellschaft

CH-4002 Basel(CH)

(72) Erfinder: Suchy, Milos, Dr.
Grossplatzstrasse 3
CH-8122 Pfaffhausen(CH)

(74) Vertreter: Lederer, Franz, Dr. et al,
Patentanwälte Dr. Franz Lederer Reiner F. Meyer
Lucile-Grahn-Strasse 22
D-8000 München 80(DE)

(54) **Optisch aktive Derivate von Propionsäure der D-Reihe, diese enthaltende Unkrautbekämpfungsmittel sowie die Verwendung dieser Derivate und Unkrautbekämpfungsmittel bei der Unkrautbekämpfung.**

(57) Die Erfindung betrifft Optisch aktive Verbindungen der D-Reihe der allgemeinen Formel

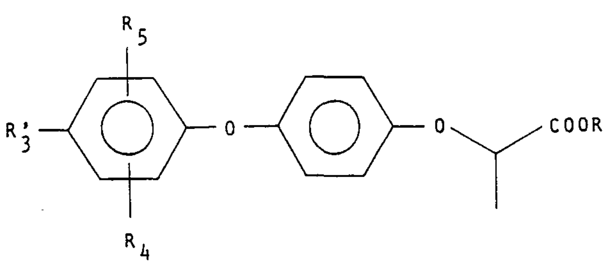

(1')

worin R Wasserstoff oder Alkyl mit 1-6 Kohlenstoffatomen, $R'_3$ Wasserstoff, Fluor, Brom, Trifluormethyl oder Nitro und $R_4$ und $R_5$ Wasserstoff oder Chlor bedeuten, mit der Massgabe, dass R nur Alkyl mit 1-6 Kohlenstoffatomen bedeutet, wenn $R_3'$ Trifluormethyl ist,
Unkrautbekämpfungsmittel, die Verbindungen der Formel I' als Wirkstoffe enthalten sowie die Verwendung dieser Verbindungen und Mittel zur Unkrautbekämpfung.

EP 0 025 079 A1

0025079

F. Hoffmann-La Roche & Co. Aktiengesellschaft, Basel, Schweiz

RAN 6102/22-001

Propionsäureester, diese enthaltende Unkrautbekämpfungsmittel sowie die Verwendung dieser Propionsäureester und Unkrautbekämpfungsmittel zur Unkrautbekämpfung.

Die Erfindung betrifft optisch aktive Verbindungen der D-Reihe der allgemeinen Formel

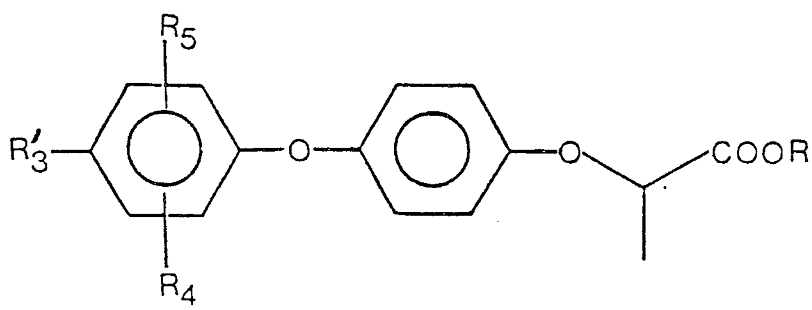

I'

worin R Wasserstoff oder Alkyl mit 1-6 Kohlenstoffatomen, $R'_3$ Wasserstoff, Fluor, Brom, Trifluormethyl oder Nitro und $R_4$ und $R_5$ Wasserstoff oder Chlor bedeuten, mit der Massgabe, dass R nur Alkyl mit 1-6 Kohlenstoffatomen bedeutet, wenn $R_3'$ Trifluormethyl ist.

BAD ORIGINAL

Die vorstehend angegebenen Verbindungen sind wertvolle Ausgangsmaterialien zur Herstellung von Verbindungen der Formel I

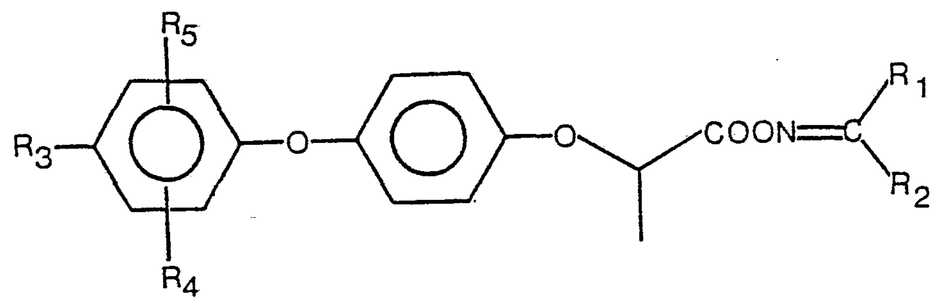

I

worin $R_1$ Wasserstoff, Alkyl mit 1-6 Kohlenstoffatomen oder Phenyl, $R_2$ Wasserstoff, Alkyl mit 1-6 Kohlenstoffatomen, Alkenyl mit 2-6 Kohlenstoffatomen, Alkinyl mit 2-6 Kohlenstoffatomen oder Phenyl oder $R_1$ und $R_2$ zusammen mit dem Kohlenstoffatom, an das sie geknüpft sind, einen Cyclohexanring bilden, der gegebenenfalls mono-, di- oder tri-Alkyl mit 1-3 Kohlenstoffatomen substituiert sein kann, $R_3$ Wasserstoff, Fluor, Chlor, Brom, Trifluormethyl oder Nitro und $R_4$ und $R_5$ Wasserstoff oder Chlor darstellen, mit der Massgabe, dass $R_1$ und $R_2$ nicht gleichzeitig Wasserstoff bedeuten., welche in der europäischen Patentanmeldung Nr. 78.101467.5 (Veröffentlichungsnummer 2.246) beschrieben werden. Die erfindungsgemässen Verbindungen der Formel I' sind zusätzlich auch als Herbicide wertvoll, da sie ein ähnliches Aktivitätsspektrum wie die Verbindungen der Formel I aufweisen. Sie besitzen den Vorteil, dass sie jedoch im Vergleich zum jeweiligen Racemat beispielsweise eine geringere Phytotoxizität auf Baumwolle und Soyabohnen aufweisen.

Von jenen Verbindungen der Formel I wird in der europäischen Patentanmeldung Nr. 78.101467.5 gesagt, dass sie sich besonders zur Bekämpfung von Ungräsern, insbesondere von Ackerfuchsschwanz (Alopecurus myosuroides) und Hirsearten wie beispielsweise Hühnerhirse (Echinochloa crus-galli), grosse Borstenhirse (Setaria faberii) und haarartige Hirse (Panicum capillare) in Getreide - insbesonders Gerste-, Hafer- und Weizen- sowie Reis-, Baumwolle-, Soya-Zuckerrüben und Gemüsekulturen eignen würden.

Besonders bevorzugte Verbindungen der Formel I' sind:

D-2-[p-(p-Bromphenoxy)phenoxy]-propionsäure,
D-2-[p-(p-Fluorphenoxy)phenoxy]-propionsäure,
D-2-[D-(p-Bromphenoxy)phenoxy]-propionsäureäthylester,
D-2-[p-(p-Nitrophenoxy)phenoxy]-propionsäure,
D-2-[p-(p-Trifluormethylphenoxy)phenoxy]-propionsäureäthylester,
D-2-[p-(p-Trifluormethylphenoxy)phenoxy]-propionsäuremethylester.

In der europäischen Patentanmeldung Nr. 78.101467.5 wird ferner gesagt, dass sich die Verbindungen der Formel I als aktive Bestandteile von herbiciden Zusammensetzungen eignen würden. Eine solche Zusammensetzung enthalte zweckmässigerweise zumindest eines der folgenden Materialien: Trägerstoffe, Netzmittel, inerte Verdünnungsmittel und Lösungsmittel.

Wenn gewünscht, könnten die Verbindungen der Formel I in einem mit Wasser nicht mischbaren Lösungsmittel, wie beispielsweise einem hochsiedenden Kohlenwasserstoff, gelöst werden, das zweckmässigerweise gelöste Emulgatoren enthalte, so dass es bei Zugabe zu Wasser als selbstemulgierbares Oel wirke.

Die Verbindungen der Formel I könnten auch mit einem Netzmittel mit oder ohne inertes Verdünnungsmittel zur Bildung eines netzbaren Pulvers vermischt werden, welches in Wasser löslich oder dispergierbar ist, oder sie könnten mit dem inerten Verdünnungsmittel zur Bildung eines festen oder pulverförmigen Produktes vermischt werden.

Inerte Verdünnungsmittel, mit welchen die Verbindungen der Formel I verarbeitet werden könnten, seien feste inerte Medien, einschliesslich pulverförmige oder feinverteilte Feststoffe, wie beispielsweise Tone, Sande, Talk, Glimmer, Düngemittel und dgl., wobei solche Produkte entweder staubförmig oder als Materialien mit grösserer Teilchengrösse vorliegen könnten.

Die Netzmittel könnten anionische Verbindungen, wie beispielsweise Seifen, Fettsulfatester, die Dodecylnatriumsulfat, Octadecylnatriumsulfat und Cetylnatriumsulfat, fettaromatische Sulfonate, wie Alkylbenzolsulfonate oder Butylnaphthalinsulfonate, komplexere Fettsulfonate, wie die Amidkondensationsprodukte von Oelsäure und N-Methyltaurin oder das Natriumsulfonat von Dioctylsuccinat sein.

Die Netzmittel könnten auch nichtionische Netzmittel wie beispielsweise Kondensationsprodukte von Fettsäuren, Fettalkoholen oder fettsubstituierten Phenolen mit Aethylenoxyd, oder Fettsäureester und -äther von Zuckern oder mehrwertigen Alkoholen oder die Produkte, die aus letzteren durch Kondensation mit Aethylenoxyd erhalten werden oder die Produkte, die als Blockcopolymere von Aethylenoxyd und Propylenoxyd bekannt sind, sein. Die Netzmittel könnten auch kationische Mittel, wie beispielsweise Cetyltrimethylammoniumbromid und dgl., sein.

Die herbicide Zusammensetzung könne auch in Form einer Aerosolverbindung vorliegen, wobei zweckmässigerweise zusätzlich zum Treibgas, welches geeigneterweise ein polyhalogeniertes Alkan wie Dichlordifluormethan sei, ein Co-Solvens und ein Netzmittel verwendet werde.

Die die Verbindungen der Formel I enthaltenden Zusammensetzungen könnten zusätzlich zu den Verbindungen der Formel I Synergisten und andere aktive Insektizide, Bakterizide, Herbicide und Fungizide enthalten.

In ihren verschiedenen Anwendungsgebieten könnten die Verbindungen der Formel I in unterschiedlichen Mengenverhältnissen eingesetzt werden. Im allgemeinen genüge eine Konzentration von 0,1 bis 6 kg/ha, vorzugsweise 0,6-2,0 kg/ha, besonders bevorzugt 1-1,5 kg/ha, um den gewünschten herbiciden Effekt zu erzielen.

Die Unkrautbekämpfungsmittel der europäischen Patentanmeldung Nr. 78.101467.5 könnten in einer Form vorliegen, die sich für die Lagerung und den Transport eignen. Solche Formen könnten z.B. 2-90 % an einem oder mehreren Wirkstoffen der Formel I enthalten. Diese Formen könnten dann mit gleichen oder verschiedenen Trägermaterialien bis zu Konzentrationen verdünnt werden, die sich für den praktischen Gebrauch eignen. Im gebrauchsfertigen Mittel könnten dann Wirkstoffkonzentrationen von 2-80 % (Gewichtsprozent) vorliegen. Die Wirkstoffkonzentration könnte jedoch auch kleiner oder grösser sein. Je nach Verwendungszweck sei eine Wirkstoffkonzentration von 2-8% bzw. 50-80% besonders bevorzugt.

Die folgenden Beispiele illustrieren die vorliegende Erfindung.

## Beispiel 1

### Herstellung von D-2-[p-(p-Trifluormethylphenoxy) phenoxy] propionsäureäthylester

50 g p-(p-Trifluormethylphenoxy)phenol werden mit 38,4 g L(-)-2-(Methylsulfonyloxy)propionsäureäthylester vermischt und danach werden 11,4 g wasserfreies Soda zugegeben. Das Gemisch wird 18 Stunden bei 110°C gerührt, dann abgekühlt und mit 300 ml Aether versetzt. Die organische Phase wird mit 2 N NaOH und Wasser ausgewaschen, über Natriumsulfat getrocknet und eingeengt. Man erhält D-2-[p-(p-Trifluormethylphenoxy)phenoxy]propionsäureäthylester; $[\alpha]_D^{22} = +19,2^o$ ($CHCl_3$; c = 1,05%).

## Beispiel 2

### Verwendung von D-2-[p-(p-Trifluormethylphenoxy)phenoxy] propionsäuremethylester als Herbicid

% Necrosis bei Baumwolle cv. Stoneville 7A

| Dosierung kg/ha | |
|---|---|
| 1,25 | 22 |
| 2,5 | 48 |

Formulierung:

| | |
|---|---|
| Wirkstoff | 250 g/l |
| N-Methylpyrrolidon | 300 g/l |
| Tensiofix B 7425 | 100 g/l |
| Phenylsulfonat CA | 25 g/l |
| Shellsol AB auf auffüllen. | 1000 ml |

Netzmittel: 0,08% Nonoxynol

Wachstumsstadium bei Behandlung: 2 Blattstadium

Sprühvolumen: 1000 l/ha

Bonitierung: 10 Tage nach Behandlung

Ort: Gewächshaus

## Patentansprüche

1. Optisch aktive Verbindungen der D-Reihe der allgemeinen Formel

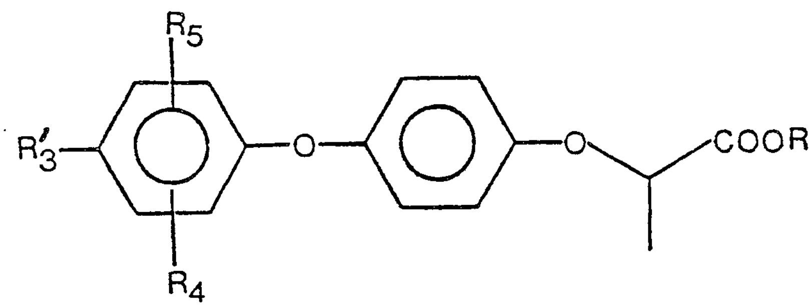

worin R Wasserstoff oder Alkyl mit 1-6 Kohlenstoffatomen, $R'_3$ Wasserstoff, Fluor, Brom, Trifluormethyl oder Nitro und $R_4$ und $R_5$ Wasserstoff oder Chlor bedeuten, mit der Massgabe, dass R nur Alkyl mit 1-6 Kohlenstoffatomen bedeutet, wenn $R_3'$ Trifluormethyl ist.

2. D-2-[p-(p-Bromphenoxy)phenoxy]-propionsäure.

3. D-2-[p-(p-Fluorphenoxy)phenoxy]-propionsäure.

4. D-2-[p-(p-Trifluormethylphenoxy)phenoxy]-propionsäureäthylester.

5. D-2-[p-(p-Trifluormethylphenoxy)phenoxy]-propionsäuremethylester.

6. D-2-[p-(p-Bromphenoxy)phenoxy]-propionsäureäthylester.

7. Eine Verbindung gemäss einem der Ansprüche 1-6 als Unkrautbekämpfungsmittel.

8. Unkrautbekämpfungsmittel, dadurch gekennzeichnet, dass es mindestens eine Verbindung der allgemeinen Formel

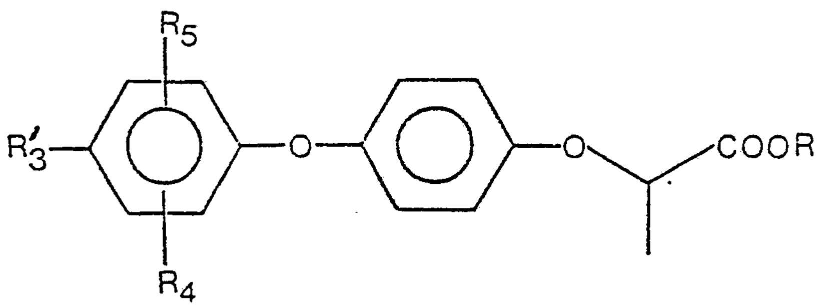

I'

worin R Wasserstoff oder Alkyl mit 1-6 Kohlenstoffatomen, $R'_3$ Wasserstoff, Fluor, Brom, Trifluormethyl oder Nitro und $R_4$ und $R_5$ Wasserstoff oder Chlor bedeuten, mit der Massgabe, dass R nur Alkyl mit 1-6 Kohlenstoffatomen bedeutet, wenn $R_3'$ Trifluormethyl ist,

und inertes Trägermaterial enthält.

9. Unkrautbekämpfungsmittel nach Anspruch 8, dadurch gekennzeichnet, dass es D-2-[p-(p-Trifluormethylphenoxy) phenoxy] propionsäure-äthylester enthält.

10. Unkrautbekämpfungsmittel nach Anspruch 8, dadurch gekennzeichnet, dass es D-2-[p-(p-Trifluormethylphenoxy) phenoxy] propionsäure-methylester enthält.

11. Verfahren zur Bekämpfung von Unkräutern, dadurch gekennzeichnet, dass man die zu schützenden Gegenstände mit einem in den Ansprüchen 8-10 genannten Mittel behandelt.

12. Verwendung der in den Ansprüchen 1-6 genannten Verbindungen als Unkrautbekämpfungsmittel.

*******

0025079

Europäisches Patentamt

# EUROPÄISCHER RECHERCHENBERICHT

Nummer der Anmeldung
EP 80 10 2316

| Kategorie | EINSCHLÄGIGE DOKUMENTE: Kennzeichnung des Dokuments mit Angabe, soweit erforderlich, der maßgeblichen Teile | betrifft Anspruch | KLASSIFIKATION DER ANMELDUNG (Int Cl ³) |
|---|---|---|---|
| X | DE - A - 2 531 643 (ISHIHARA SANGYO KAISHA)<br>* Ansprüche 1,12; Seite 10, Verbindung Nr. 1 *<br>-- | 1,7,8, 11,12 | C 07 C 59/68<br>69/712<br>79/35<br>A 01 N 31/16 |
| P | FR - A - 2 387 937 (HOFFMANN-LA ROCHE)<br>* Seite 5, Zeile 31 bis Seite 6, Zeile 2; Seite 8, Zeilen 1-15; Ansprüche 13-15 *<br>-- | 1,7,8 11,12 | |
| | DE - A - 2 433 067 (HOECHST)<br>* Ansprüche 1,3-5 *<br>-- | 1,4,5, 7-12 | RECHERCHIERTE SACHGEBIETE (Int Cl ³)<br>C 07 C 59/68<br>69/712 |
| | FR - A - 2 000 174 (HOECHST)<br>* Seite 5, Zeilen 26,32 *<br>-- | 2,3 | |
| | NL - A - 73 06625 (HOECHST)<br>* Anspruch 1; Seite 13, Verbindung LXII; Seite 15, Verbindungen LXXXIX, XCI *<br>& FR - A - 2 184 906<br>& DE - A - 2 223 894<br>-- | 1,2,6-8,11, 12 | KATEGORIE DER GENANNTEN DOKUMENTE<br>X von besonderer Bedeutung<br>A technologischer Hintergrund<br>O nichtschriftliche Offenbarung<br>P Zwischenliteratur<br>T der Erfindung zugrunde liegende Theorien oder Grundsätze<br>E kollidierende Anmeldung<br>D in der Anmeldung angeführtes Dokument<br>L aus andern Gründen angeführtes Dokument |
| E | EP - A - 0 002 246 (HOFFMANN-LA ROCHE)<br>* Ansprüche 22-27; Seite 10, Zeile 25 bis Seite 11, Zeile 22 *<br>---- | 1-12 | & Mitglied der gleichen Patentfamilie, übereinstimmendes Dokument |

X Der vorliegende Recherchenbericht wurde für alle Patentansprüche erstellt.

| Recherchenort | Abschlußdatum der Recherche | Prüfer |
|---|---|---|
| Den Haag | 05-11-1980 | KLAG |

EPA form 1503.1 06.78